# EUROPEAN PATENT APPLICATION

(11) **EP 4 738 382 A1**
(43) Date of publication of application: **06.05.2026**
(21) Application number: 24210950.2
(22) Date of filing: 05.11.2024
(51) Int. Cl.: G16H 40/63

(54) **HANDS-FREE MEDICAL DEVICE CONTROL**

(71) Applicant: Koninklijke Philips N.V., 5656 AG Eindhoven (NL)
(72) Inventor: BULUT, Murtaza, Eindhoven (NL); VAN LIESHOUT, Ron Martinus Laurentius, Eindhoven (NL); HUIJBREGTS, Laurentia Johanna, 5656AG Eindhoven (NL); VAN DER HEIDE, Esther Marjan, Eindhoven (NL); NIKOLAYEV, Aleksander, Eindhoven (NL)
(74) Representative: Philips Intellectual Property & Standards

(57) **Abstract**

The present disclosure provides concepts for detecting a hands-free input from a user for controlling a medical device. Specifically, an occurrence of an alert triggered by the medical device is identified, and the hands-free input is detected from the user. The detection of the hands-free input is based in part on one or more characteristics of the alert. Thus, more accurate and context-aware detection of hands-free inputs in medical environments is achieved by leveraging characteristics (e.g., timing, origin, urgency, etc.) of an alert generated by the medical device, improving the reliability and efficiency of medical device control in medical environments.

## Description

### FIELD OF INVENTION

The present invention relates to hands-free control of medical devices, and more particularly to systems and methods for detecting hands-free input from a user for controlling a medical device.

### BACKGROUND

Patient monitoring systems are widely used in healthcare settings to continuously track vital signs and alert medical staff to potential issues. These systems typically generate alerts (e.g., alarms, notifications, communication requests, etc.) when measured parameters fall outside of preset ranges. However, the high volume of alerts produced by modem monitoring equipment has led to challenges for healthcare providers. Indeed, a wide range of medical devices beyond just patient monitors now utilize alerts to attract the attention of medical staff. Studies have shown that up to 85-99% of alerts may not require clinical intervention, contributing to alert fatigue among medical staff.

Responding to and managing alerts consumes a significant portion of clinicians' time and attention. Current alert management typically requires manual interaction with the medical device to silence, acknowledge, or adjust alert settings. This process can be disruptive to workflow and patient care, especially in busy clinical environments. Additionally, the need for direct physical contact with medical devices may increase the risk of contamination in sensitive healthcare settings.

It has been appreciated that an improved means for interacting with medical devices is needed that overcomes one or more of these problems.

### SUMMARY OF INVENTION

The invention is defined by the claims.

According to an aspect of the present disclosure, a method for detecting a hands-free input from a user for controlling a medical device is provided. The method comprises identifying an occurrence of an alert triggered by the medical device, and detecting the hands-free input from the user, wherein detection of the hands-free input is based in part on one or more characteristics of the alert.

The present disclosure provides concepts for detecting a hands-free input from a user for controlling a medical device. Specifically, an occurrence of an alert triggered by the medical device is identified, and the hands-free input is detected from the user. The detection of the hands-free input is based in part on one or more characteristics of the alert. Thus, more accurate and context-aware detection of hands-free inputs in medical environments is achieved by leveraging characteristics (e.g., timing, origin, urgency, etc.) of an alert generated by the medical device, improving the reliability and efficiency of medical device control in medical environments.

Proposed concepts thus aim to provide schemes, solutions, concepts, designs, methods, and systems pertaining to hands-free control of medical devices (e.g., subject monitors), particularly in the context of alert management. In particular, embodiments aim to provide improved methods and systems for detecting hands-free input from users to control medical devices based on alert characteristics.

The invention presents a novel approach to hands-free medical device control that leverages the context related to alert events. By identifying the occurrence of an alert triggered by a medical device and detecting hands-free input from a user based in part on characteristics of the alert, the system can more accurately interpret user intentions and actions. This context-aware approach to hands-free input detection can significantly improve the reliability and efficiency of medical device control in potentially noisy or complex healthcare settings.

A key advantage of the proposed method is its ability to adapt to different alert scenarios. The detection of user actions can be initiated in response to an alert, with optional delays based on factors like expected review time or alert audio length. This flexibility allows the system to align with natural user behaviors and workflows. Furthermore, the detection window and sensor parameters can be dynamically adjusted based on alert characteristics, optimizing the system's performance for each specific situation. In addition, the interpretation of the user actions (e.g., gestures, speech, etc.) and translation of user actions into the hands-free input for control of the medical device, may be adjusted based on the alert characteristics to further improve accuracy of control.

By considering a wide range of alert attributes (e.g., timing, urgency, source), the proposed method offers a comprehensive and nuanced approach to hands-free medical device control. This holistic consideration of context has the potential to significantly enhance the user experience, improve efficiency, and reduce errors in critical healthcare environments.

In some embodiments, the method may further comprise detecting an action of the user based on the characteristics of the alert. Basing the detection of user actions on alert characteristics can lead to more accurate, reliable and efficient detection/measurement of user actions from which the hands-free input can be derived.

More specifically, the detection of an action of the user may be initiated responsive to the alert being triggered. This feature ensures that the system is actively looking for user inputs responsive to alerts when they are most likely to occur, improving response times and reducing unnecessary processing. Indeed, it is typically the case that trigger words/actions are used to prompt systems to begin detection of hands-free input related user actions. In this case, the alert being triggered is instead used as the trigger for detection of the input from the user, reducing cognitive and practical load on the user whilst ensuring the detection is only performed when necessary.

In some cases, the initiation of detection of the action from the user may be delayed for a predetermined length of time. The predetermined length of time may be based on at least one of an expected time to review the alert by the user, an expected time for a user to observe the medical device, a length of an output associated with the alert, and/or a user preference.

Delaying the initiation of action detection allows for more natural user interactions and can reduce false detections that might occur immediately after an alert is triggered. Indeed, it is likely that a user will be in a position to provide an input (or at least a well-thought through input) immediately after an alert is triggered.

Furthermore, the detection of the action of the user may be performed during a detection window, wherein the length of the detection window is based on the characteristics of the alert. Adjusting the detection window based on alert characteristics allows for more efficient use of system resources and can improve the accuracy of hands-free input detection.

In addition, the detection of the action of the user may be performed using one or more selected sensors operated according to one or more operation parameters. This feature allows for flexible and optimized sensor usage based on the specific alert and environmental conditions, potentially improving detection accuracy and efficiency.

In some embodiments, the method may further comprise detecting the location of the user relative to the medical device. In this case, detection of the hands-free input is further based on the detected location of the user.

Incorporating user location information can significantly improve the accuracy of hands-free input detection by considering the spatial context of user actions. Furthermore, it is important to establish that the user is present in order to properly detect the hands-free input.

To be more specific, the detection of the hands-free input from the user may comprise processing, with an action recognition algorithm, a detected action of the user to determine the hands-free input. In this case, the processing is based on the characteristics of the alert.

In other words, the actions of the users (detected or measured by various sensors) are interpreted, filtered and analyzed to determine the intent of the user in providing the hands-free input. This approach allows for more sophisticated interpretation of user actions, taking into account the context provided by the alert characteristics to improve input recognition accuracy.

In some embodiments, the processing of the detected action of the user based on the characteristics of the alert may comprise modifying the action recognition algorithm based on the characteristics of the alert, and processing, with the modified action recognition algorithm, the detected action of the user.

By dynamically modifying the action recognition algorithm based on alert characteristics, the system can adapt to different scenarios and potentially improve recognition accuracy across a wide range of situations. Indeed, it may be important to modify the interpretation, analysis and filtering of the user action in light of the alert characteristics to accurately determine the intended hands-free input of the user.

The modification of the action recognition algorithm may comprise at least one of increasing or decreasing a sensitivity of the action recognition algorithm to user actions, and/or altering a mapping between detected user actions and hands-free inputs by the action recognition algorithm. This flexibility in algorithm modification allows for fine-tuned adjustments to the recognition process, potentially reducing errors and improving the overall user experience.

In addition, the processing of the detected action of the user based on the characteristics of the alert may comprise filtering the detected action based on the characteristics of the alert, and processing, with the action recognition algorithm, the filtered detected action of the user.

Filtering detected actions based on alert characteristics can help reduce noise and irrelevant inputs, potentially improving the accuracy and efficiency of the hands-free input detection process.

The processing, with the action recognition algorithm, of the detected action of the user to determine the hands-free input may be further based on one or more characteristics of the user.

Incorporating user characteristics into the processing can allow for more personalized and accurate interpretation of user actions, potentially improving the overall performance of the hands-free input detection system.

In specific embodiments, the one or more characteristics of the alert may include an alert timing, an alert origin, an alert urgency, an alert urgency trend, an alert trigger, an alert location, an alert source, alert transfer data, an alert output medium characteristic, an expected user action associated with the alert, and an alert circumstance.

Considering a wide range of alert characteristics allows for a more comprehensive and nuanced approach to hands-free input detection, potentially improving accuracy across diverse medical scenarios.

According to a further aspect of the present disclosure, a computer program is provided. The computer program comprises computer program code means adapted, when said computer program is run on a computer, to implement a method for detecting a hands-free input from a user for controlling a medical device as described herein.

According to another aspect of the present disclosure, a controller for a medical device is provided. The controller comprises a monitoring system configured to identify an occurrence of an alert triggered by the medical device, and a hands-free interface configured to detect a hands-free input for controlling the subject-monitor from a user, wherein detection of the hands-free input by the hands-free interface is based in part on one or more characteristics of the alert.

These and other aspects of the invention will be apparent from and elucidated with reference to the embodiment(s) described hereinafter.

### BRIEF DESCRIPTION OF FIGURES

For a better understanding of the invention, and to show more clearly how it may be carried into effect, reference will now be made, by way of example only, to the accompanying drawings, in which:
FIG. 1 illustrates a flowchart for a method of detecting hands-free input for controlling a medical device, according to aspects of the present disclosure;
FIG. 2 illustrates a timeline of events related to alert management and action detection, according to an embodiment;
FIG. 3 illustrates a block diagram of a medical device control system, in accordance with example embodiments; and
FIG. 4 is a simplified block diagram of a computer within which one or more parts of an embodiment may be employed.

### DETAILED DESCRIPTION

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Proposed concepts thus aim to provide schemes, solutions, concepts, designs, methods, and systems pertaining to hands-free control of medical devices, particularly in the context of alert management. In particular, embodiments aim to provide improved methods and systems for detecting hands-free input from users to control medical devices based on alert characteristics.

More specifically, the present disclosure provides methods and systems for hands-free control of medical devices, particularly in the context of alert management. The disclosed methods and systems aim to improve the efficiency and accuracy of medical device control in healthcare environments, which can be complex and noisy. The disclosed methods and systems are designed to detect hands-free input from a user, such as a healthcare provider, for controlling a medical device, such as a subject monitor.

The invention presents a novel approach to hands-free medical device control that leverages the context provided by alert events. By identifying the occurrence of an alert triggered by a medical device and detecting hands-free input from a user based in part on characteristics of the alert, the system can more accurately interpret user intentions and actions. This context-aware approach to hands-free input detection can significantly improve the reliability and efficiency of medical device control in potentially noisy or complex healthcare settings.

A key advantage of the proposed system is its ability to adapt to different alert scenarios. The detection of user actions can be initiated in response to an alert, with optional delays based on factors like expected review time or alert audio length. This flexibility allows the system to align more naturally with user behaviors and workflows. This contrasts with typical systems, that usually require trigger actions (e.g., trigger words or trigger gestures) in order to the hands-free input detection to be initiated. Alternatively, systems may always detect the hands-free user, which is computationally expensive and has associated privacy concerns.

Furthermore, the detection window and sensor parameters can be dynamically adjusted based on alert characteristics, optimizing the system's performance for each specific situation. Specifically, the sensitivity and specificity of the detection of hands-fee user inputs may be adjusted depending on the scenario indicated by the alert characteristics, for more accurate and reliable detection.

The invention also incorporates sophisticated processing of detected user actions. By modifying action recognition algorithms based on alert characteristics, the system can fine-tune its interpretation of user inputs. This may involve adjusting sensitivity to certain actions or altering the mapping between detected actions and device controls. Additionally, the system can filter detected actions based on alert context, potentially reducing false positives and improving overall accuracy.

The detection of the hands-free input is based, at least in part, on one or more characteristics of an alert triggered by the medical device. These characteristics may include, but are not limited to, alert timing, alert origin, alert urgency, alert trigger, alert location, alert source, alert output medium characteristic, an expected user action associated with the alert, and alert circumstance. This holistic consideration of context has the potential to significantly enhance the user experience, improve efficiency, and reduce errors in critical healthcare environments.

The proposed invention may be particularly suited for controlling subject monitors (whether for one subject, or a central monitoring station for one or more subjects). Indeed, subject monitors output alerts regularly to attract the attention of healthcare professionals to changes in the state and condition of the monitored subject(s). Users must therefore regularly interact with subject monitors to silence, turn off, reconfigure, and/or perform an action in relation to the alert. It is therefore particularly useful to gather a hands-free input for controlling such subject monitors. By leveraging information associated with the alert triggered by the subject monitor (e.g., timing, urgency, etc.), the detection of the hands-free input may be adapted to be more accurate, reliable etc. Nevertheless, it is of course the case that other medical devices (e.g., infusion pumps, ventilators) may be configured to trigger an alert (or have an alert triggered on behalf of said medical device), and therefore this invention may equally apply to those medical devices.

Furthermore, the invention may particularly benefit the detection of a speech input from the user. Speech is an ideal mode for interacting with medical devices, as it enables the medical professional to perform other (potentially very important) actions with their hands, whilst also reducing the risk of contamination spread via touch, and obviating the need to remove gloves, for example. Furthermore, with the increasing use of speech enabled personal devices, users are becoming increasingly familiar with the use of speech input. Nevertheless, it is of course the hands-free input may be performed in other (or a mix of) modes, such as hand gestures, gaze tracking, etc. In any case, the present invention may be applied to any input mode that does not require the user to touch a physical user interface.

In other words, speech is a hands-free input mode that particularly benefits from the present invention. Although speech recognition accuracy has considerably improved by virtue of deep learning models, in environments where there is continuous noise coming from different devices and people (e.g., such as hospital ICUs), the speech recognition accuracy may suffer. When controlling medical devices, such as subject monitors, an accuracy/sensitivity/specificity near 100% is necessary, and even small errors may be unacceptable and potentially dangerous. Nevertheless, due to the sheer quantity of alerts and the fast-paced environment in which they occur, utilizing speech input for management of the alerts can significantly improve the efficiency of the caregivers.

Thus, considering that most interactions with the medical devices (and particularly subject monitors) are for alert management, it has been realized that it is beneficial to directly using alert-specific characteristics and information to modify the detection hands-free inputs. Whilst this is primarily an advantage for speech inputs, the same logic can be applied to gesture and eye tracking based interactions.

Embodiments of the proposed invention therefore perform detection of the hands-free input based on alert characteristics. More specifically, specific preselected settings of action detection systems are modified depending on the alert characteristics. Specifically, the focus is on the generated alerts. The alert information from the medical device (e.g., patient monitor, ventilator) as well as information from the nearby medical devices (e.g. patient monitor of a second patient) may be considered. That is, by making use of the time related information, type, and underlying reason for the generated alerts, the settings of the action detection algorithm (e.g., speech recognition algorithm) and action detection/measurement means are adjusted. The focus is on adjusting settings related to input collection (i.e., action detection), input processing (e.g., action recognition, analysis and interpretation), input translation recognition, and action filtering.

In the case that the hands-free input is a speech input, the following can be considered when adjusting the speech detection based on the alert characteristics:
(i) Speech detection, or speech collection settings. This may relate to the initialization and duration of speech detection based on alert characteristics. For example, the speech detection may begin responsive to the alert being triggered, or after a short delay. The speech detection duration may depend on how long it is expected for a user to be able to react to the alert. Further this may involve changing the settings and operating parameters of the microphone setup used to capture the speech of the user.
(ii) Speech recognition settings, or sound-to-text settings. This may relate to the adjustment of sensitivity of the detection algorithm to certain words or phrases, or the sensitivity toward speech of certain individual users based on characteristics of the alert.
(iii) Input translations/interpretation settings. The understanding and translation of speech that has been collected and recognized to actions that can be performed on the medical device (i.e., actual inputs to the medical device) may be adapted based on the type, urgency, timing etc. of the alert. Furthermore, user-related characteristics (e.g., typical user style and vocabulary) may also be considered.
(iv) Noise filtering settings. Specifically, the noise produced by the alert may be considered when collecting the speech of the user (whether detected or pre-learned).

Of course, the same logic as the above may be applied to other hands-free input nodes.

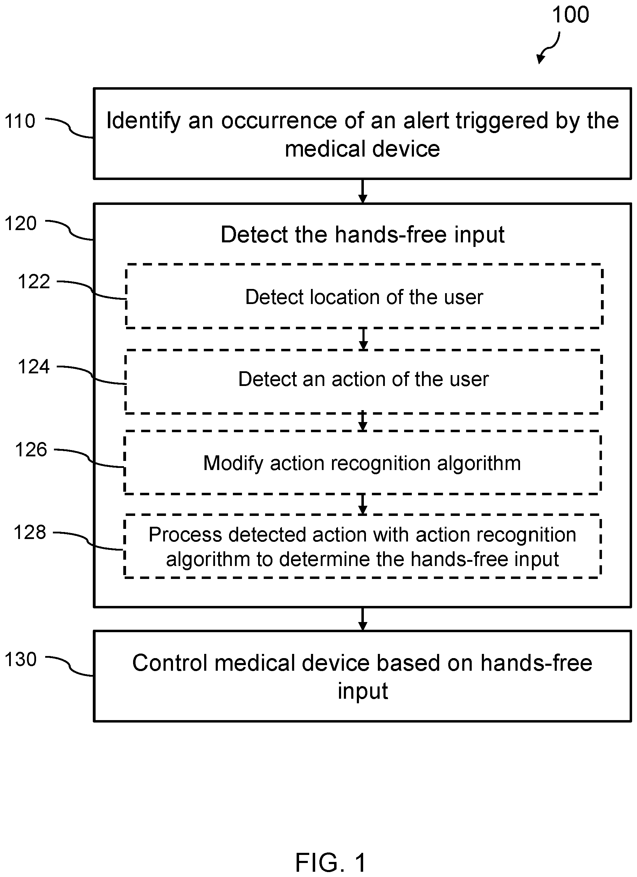

Referring now to FIG. 1, there is depicted a flow diagram of a method 100 for detecting a hands-free input from a user for controlling a medical device. As stated above, the hands-free input may be, for example, a speech input, a gesture input, an eye gaze input, or any other input that is remote from a physical user interface. The medical device is associated with an alert being triggered (which may be performed by the medical device itself, or by another device on behalf of the medical device). For example, the medical device may be a personal subject monitor, a centralized subject monitor, a ventilator, or an infusion pump.

The method 100 begins in step 110 with the occurrence of an alert triggered by the medical device being identified. This identification process may involve monitoring the medical device for any alert signals or alerts. To this end, the monitoring of the medical device may be performed by a dedicated sensor, or an indication of the alert may be received from the medical device itself.

The alert signals may be generated due to various conditions or events, such as a change in a subject's vital signs, a technical issue with the medical device, or a pre-set alert trigger. Alerts may be provided in the form of any sensory output that attempts to attract the attention of the user. For example, the alert may be an alarm. Furthermore, the alert may be a notification, indication or other request, with the aim of prompting a user (e.g., a caregiver) to either receive new information (e.g., that certain vital signs have changed, that the subject has moved/left a bed, that the environment has changed, or that an algorithm has provided an updated estimation), or to provide information to a system (e.g., enter information about the patient, environment, etc.).

The alert may have various characteristics. The alert characteristics may include an alert timing, which indicates when the alert began and stopped (both past and present). Indeed, the alarm may have previously been silenced, and subsequently re-triggered. The alert may have an associated urgency, such as low, medium or high, or an associated color representing the urgency. That is, the alert may be associated with a given timeframe for a medical professional to react to the alert. The alert may have an associated alert trigger, being the reason for the alert to be triggered (e.g., a vital sign exceeding a threshold, a fault being detected, etc.). The alert may have an associated urgency trend, being a change in the urgency over time (and potentially an expected future change in urgency). As a result, the characteristic may reflect an escalation or de-escalation in the urgency of the alert. The alert may have an associated source, being the condition and/or reason for the alert to be triggered. The alert may have associated alert transfer data reflecting how the alert has been transmitted between different systems and/or between different intended users, and any communications related to said transfer. The alert may have an output medium characteristic. The output medium characteristic may describe the means by which the alert presents/is realized (e.g., sound, haptic, light, etc.), or the features of the alert being presented (e.g., a frequency, a magnitude/loudness, etc.). The alert may also be associated with an expected user action, describing the expected action (or set of actions) that a user may be expected to take responsive to being made aware of the occurrence of the alert (e.g., expect that the healthcare professional takes manual vital signs of a subject, or replaces a faulty lead, etc.). The alert may have an associated circumstance/environmental condition (e.g., other alerts occurring simultaneously). Of course, other characteristics of the alert may be considered.

Thus, identifying the occurrence of the alert may also comprise obtaining and/or determining one or more of the above characteristics of the alert.

Following the identification of the alert, the method 100 proceeds to detecting 120 the hands-free input from the user. To reiterate, the hands-free input may be in the form of speech, gestures, eye movements, or any other form of input that does not require the user to physically interact with the medical device. The detection of the hands-free input is based in part on the one or more characteristics of the alert. That is, the process of detection (when to initiate detection, how to perform detection, and how to process and interpret the users' action to determine the user input) is based upon the characteristics (e.g., timing, urgency, mode, etc.) of the alert.

The detection of the hands-free input may be performed using any known means. That is, any sensor and processing apparatus known to be able to measure and interpret user actions to determine a user input may be utilized. Such options are well known and understood by the skilled person.

In some aspects, the detection of the hands-free input from the user involves sub-step 122 in which the location of the user relative to the medical device is detected. This may be achieved using any known means. In this case, detection of the hands-free input is further based on the detected location of the user.

To be specific, the detection of the hands-free input of the user may not be performed until the user is nearby the medical device. This is so that the input is only detected once the user has been able to process the alert. Indeed, there is no point in receiving the user input if the user is not in a position to provide the hands-free input.

Furthermore, the detection of the hands-free input from the user may be dependent on the location of the user, in that the means and way of detecting the input may be adjusted based on the location of the user. Specifically, if the user is near certain sensors for detection of the input, those sensors may be prioritized over sensors that are far away from the user.

There may also be provided sub-set 124. In sub-step 124, an action of the user is detected/measured. That is the actual speech, and/or movement of the user is detected/measured, from which the hands-free user input may be derived. This action detection may be based on the characteristics of the alert. For example, the time window of detection of the action may be dependent on the alert characteristics (e.g., when the alert occurred, for how long, etc.), or the sensors and their operation parameters (e.g., sensitivity, directivity, etc.) may be altered based on the alert characteristics.

More specifically, when the detection of the action of the user 124 is performed during a detection window (i.e., a time window during which detection is performed), the length of the detection window is based on the characteristics of the alert. Some alerts may only require a fast, quick response and therefore an input may only be received during a small detection window. Alternatively, the alert may require a more complex and considered interaction, and therefore an input may require a larger time window to be properly detected.

In some cases, detecting an action of the user may simply be initiated responsive to the alert being triggered by the subject monitor. In other words, the alert acts as a trigger for the detection of the action from which the input is derived. In many ways, this means that the alert acts like the utterance of a known trigger word for smart home devices. In this case, the characteristic of importance of the alert is the timing of the alert.

Initiating detection of the action from the user may be delayed for a predetermined length of time. This may allow the user to properly process the meaning of the alert, and to decide how to respond to the alert, before detecting the action for the input. the predetermined length of time is based on at least one of an expected time to review the alert by the user, an expected time for a user to observe the subject monitor, a length of an output associated with the alert (e.g., length of time that noise, vibration or light is output), and/or a user preference.

Once an action is detected, the detected action may be processed by an action recognition algorithm to determine the hands-free input. In other words, the detected action needs to be filtered, analyzed, and interpreted to deduce the intended meaning of the user action and how that translates to a certain input for the medical device. For example, when the action is the user speaking, the speech of the user is detected. The detected speech may be filtered (to remove environmental noise), analyzed and converted to text, and then the text interpreted and translated to a certain input for the medical device (note, in some cases the detected speech may be directly translated to a certain input). This may be performed by a voice recognition algorithm, for example.

In other words, the method 100 may also involve processing 128 the detected action of the user with an action recognition algorithm to determine the hands-free input. The action recognition algorithm may be a machine learning model, a pattern recognition algorithm, or any other type of algorithm capable of interpreting the user's action and translating it into a command for controlling the medical device. The processing of the detected action is based on the characteristics of the alert. This may involve adjusting the parameters of the action recognition algorithm based on the type, urgency, source, or other characteristics of the alert. For example, the algorithm may be adjusted to be more sensitive to certain commands or actions that are commonly associated with a specific type of alert.

In some embodiments there is provided sub-step 126. In sub-step 126, the action recognition algorithm is modified based on the characteristics of the alert. This may involve increasing or decreasing the sensitivity of the action recognition algorithm to user actions, and/or altering a mapping between detected user actions and hands-free inputs by the action recognition algorithm. For example, if a certain user input is expected in response to the alert (deduced from known characteristics of the alert), then sensitivity to certain user actions and/or a mapping of certain actions to the expected input may be altered to make detection of such inputs more probable. Further, if the alert is of a high urgency, the algorithm may be adjusted to prioritize actions that correspond to immediate response measures.

That is, in some cases, the modification step 126 of the action recognition algorithm may involve increasing or decreasing the sensitivity of the action recognition algorithm to user actions. This adjustment can be based on the characteristics of the alert. For instance, if the alert indicates a critical condition, the sensitivity of the algorithm to user actions may be increased to ensure that all relevant actions are detected and processed. Conversely, if the alert indicates a non-critical condition, the sensitivity of the algorithm to user actions may be decreased to reduce the likelihood of false positives.

In some embodiments, the modification step 126 of the action recognition algorithm may also involve altering a mapping between detected user actions and hands-free inputs by the action recognition algorithm. This alteration can be based on the characteristics of the alert. For example, if the alert is triggered by a specific condition, the mapping may be altered to prioritize actions that are commonly associated with responding to that condition. This can ensure that the most relevant actions are recognized and processed first, potentially improving the efficiency of the hands-free control of the medical device.

Furthermore, the detected action may be filtered based on the characteristics of the alert. This filtering process may involve removing or reducing noise or irrelevant actions, thereby focusing on the actions that are most relevant to the alert. For example, if the alert is triggered by a specific condition, the filtering process may prioritize actions that are commonly associated with responding to that condition. This can help to reduce the likelihood of false positives and improve the accuracy of the hands-free control system.

For example, if the alert occurs at a certain time and produces a certain noise, then that noise may be filtered out of speech detected from the user. Furthermore, if the alert is typically associated with certain hand gestures of the user (because they need to perform certain actions in relation to a subject, for example), then those detected movements may be disregarded for the detection of a gesture-related input.

Then, in sub-step 128, the detected (and potentially filtered) action is processed with the (potentially modified) action recognition algorithm, to determine the hands-free input from the user.

Of course, this determination may be further based on one or more characteristics of the user. For instance, the user's role, experience level, or personal preferences may be considered when processing the detected action. This can allow for more personalized and efficient control of the medical device, potentially improving the user's experience and the overall effectiveness of the hands-free control system. For example, if the user has a certain way of speaking, then determination of the voice input from the user may take this into account. Furthermore, if the user has a certain role, then determination of the hands-free input may take this into account.

Furthermore, the method may take into consideration a plurality of users and their associated characteristics. That is, multiple users may be interacting with the subject, medical device, and each other in response to an alert. The characteristics of each of these users may be considered when determining the hands-free input from a user. More specifically, the role, experience level, and personal preferences of each user may be considered when processing the detected action to determine the hands-free input.

In yet another embodiment, the detection of the hands-free input of the user may take into account alerts from other medical devices in the vicinity of the medical device in question. For example, it is often the case in medical environments that multiple subject monitors are provided in relatively close proximity to each other. Each of these subject monitors may produce alerts, and may receive hands-free inputs. Indeed, the alerts produced by the subject monitors may occur at the same time, which could lead to confusion both for users and for the systems interpreting said inputs.

Accordingly, it is proposed to also consider alerts produced by nearby medical devices (e.g., other medical devices associated with a single subject, or with other subjects) in the detection of the hands-free input for the medical device in question. In one example, the timing and duration of a detection window of the action for said medical device may take into account the characteristics of the alert triggered by another medical device. More specifically, to avoid confusion arising during detection of the hands-free input, the detection window during of said medical device may be delayed until actions linked to the alert of the other medical devices are complete. For sake of clarity, users may be provided with a notification indicating that one medical device is receptive to the hands-free input, whilst the other is not. For example, an LED can be turned on to indicate that the medical device is receptive to the hands-free input. Of course, during this period, each medical device may still receive other forms of input (e.g., touch input), as these inputs may be unambiguous.

Furthermore, other aspects of detection of the hands-free input for the medical device may be adapted based on information from nearby medical devices. For example, the alert from one medical device may be delayed until the alert from other medical devices are complete for a predetermined time. In addition, the location of the user may be taken into account to determine which medical device should detect (i.e., be receptive to) the hands-free input. Also, in the case that the alert from a nearby medical device produces a known noise, and the hands-free input is a voice input, then the input to the medical device may be filtered based on the known noise. Of course, many other aspects of the nearby medical device alerts may be taken into consideration.

Finally, in step 130, the medical device is controlled based on the hands-free input. Due to the detection of the hands-fee input being dependent on the alert characteristics, the hands-free input may be more reliably and accurately detected, whilst reducing a cognitive load on the user and minimizing the processing required by the detection system.

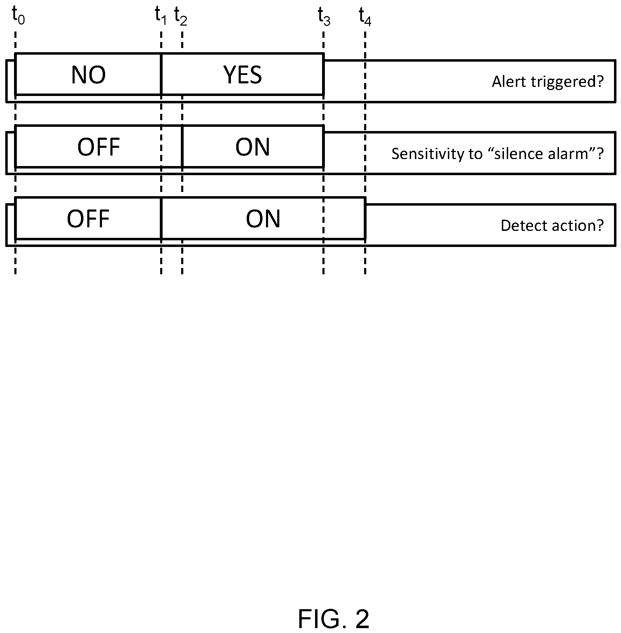

Referring to FIG. 2, an example of detection of a hands-free user input according to an embodiment of the invention is depicted.

Specifically, the timeline illustrates how the system adapts speech detection settings based on characteristics of the alert, in this case the alert timeline. At time t1, the alert becomes active (YES), but the system does not immediately start increasing its detection sensitivity to a "silence alert" command but does start detecting actions. This indicates that the system is now ready to detect and respond to alert-related actions from the user but does install a delay before the system becomes receptive to alert silencing commands. This delay may be a predetermined length of time, which may be based on an expected time to review the alert by the user, an expected time for a user to observe the medical device, a length of an output associated with the alert, and/or a user preference.

Of course, delaying of the change of sensitivity to a certain input is only one example. This concept may be applied to other aspects of the hands-free input detection. For example, translation (i.e., understanding) of user actions into the intent of the user may be delayed, or translation of the recognized intent of the user into an actual input for controlling the medical device may be delayed.

At time t2, while the alert is still active, the sensitivity to "silence alert" switches to ON. The alert remains active (YES) from t2 through t3. During this period, both the sensitivity to "silence alert" and action detection remain ON. Beyond t3, detection of the action carries on.

The length of the detection window may be set such that a user can perform potential alert related adjustments such as changing alert limits. The length of the detection window may be predetermined, or may be dependent on alert characteristics, a user profile, a user location, action dependent, or dependent on the status of the subject associated with the medical device.

That is, in some aspects, the detection of the action of the user is performed during a detection window, wherein the length of the detection window is based on the characteristics of the alert. These characteristics may include, but are not limited to, alert timing, alert origin, alert urgency, alert trigger, alert location, alert source, alert output medium characteristic, an expected user action associated with the alert, and alert circumstance. The length of the detection window may be adjusted based on these characteristics to ensure that the system is receptive to user actions during the most relevant periods.

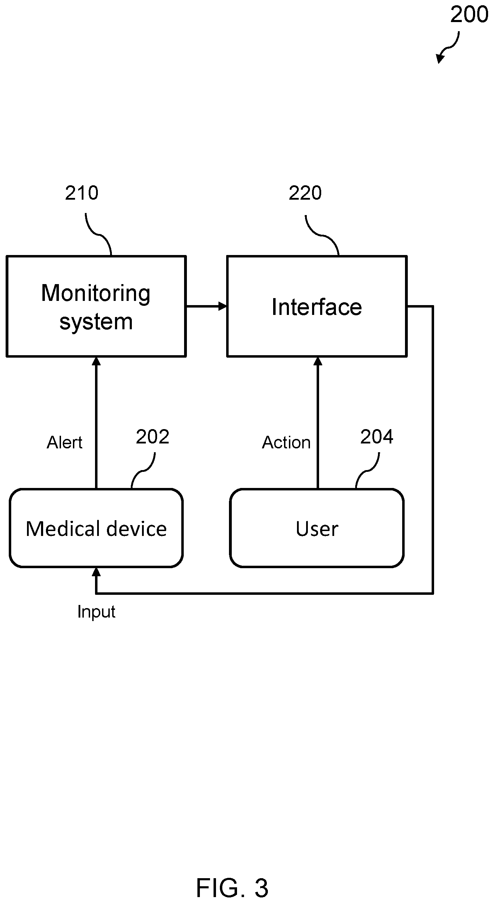

Referring to FIG. 3, a controller for a medical device control system 200 is illustrated. The controller comprises a monitoring system 210, and a hands-free input interface 220, which interact with the medical device 202 and the user 204. The medical device 202 may be any type of device used in a healthcare setting, such as a patient monitor, ventilator, infusion pump, or the like. The medical device 202 may further include any device and/or system used to control the example devices, such as a mobile device, a central monitoring station, or a tele-health system. That is, any device/interface that may be used to control alerts generated in relation to medical devices 202 may be controlled by the proposed system. The medical device 202 is configured to trigger alerts based on various conditions or events, such as changes in a patient's vital signs, technical issues with the device, or pre-set alert triggers.

The monitoring system 210 may be communicatively coupled to the medical device 202 and is configured to identify an occurrence of an alert triggered by the medical device 202. The monitoring system 210 may include various components and modules for processing and analyzing the alert signals from the medical device 202, and thus for determining or receiving alert characteristics. In some aspects, the monitoring system 210 may also be configured to monitor other parameters or data from the medical device 202, such as patient data, device status, and the like.

The hands-free interface 220 is also communicatively coupled to the monitoring system 210 and the medical device 202. The hands-free interface 220 is configured to detect a hands-free input from the user 204 for controlling the medical device 202. The hands-free input may be in the form of speech, gestures, eye movements, or any other form of input that does not require the user 204 to physically interact with the medical device 202. The detection of the hands-free input by the hands-free interface 220 is based in part on one or more characteristics of the alert identified by the monitoring system 210.

In some cases, the hands-free interface 220 may include one or more sensors for detecting the action of the user 204. These sensors may be selected and operated according to one or more operation parameters. For example, the sensors may include microphones for detecting speech, cameras for detecting gestures or eye movements, or other types of sensors suitable for detecting hands-free input. The operation parameters of the sensors (e.g., directionality, sensitivity, selectivity, etc.) may be adjusted based on the characteristics of the alert, such as the type, urgency, source, or other characteristics of the alert.

That is, the hands-free interface 220 may dynamically select and operate the sensors based on the characteristics of the alert and the detected location of the user 204. For example, if the alert is triggered by a specific condition and the user 204 is located close to the medical device 202, the system 200 may select and operate the sensors that are most suitable for detecting actions commonly associated with responding to that condition in close proximity. This dynamic sensor selection and operation can provide a more adaptive and context-aware control of the medical device 202, potentially improving the user's experience and the overall effectiveness of the hands-free control system 200.

In some embodiments, the interface 220 may also include a location detection module for detecting the location of the user 204 relative to the medical device 202. This location information may be used to further refine the detection of the hands-free input by the hands-free interface 220. For instance, if the user 204 is located close to the medical device 202, the system 200 may prioritize inputs that are commonly associated with close proximity interactions. Conversely, if the user 204 is located farther away from the medical device 202, the system 200 may prioritize inputs that are commonly associated with remote interactions. This location-aware detection can provide a more accurate and context-aware control of the medical device 202, potentially improving the efficiency and effectiveness of the hands-free control system 200.

In some aspects, the hands-free interface 220 may adapt its input understanding settings based on the type of alert, user characteristics, and context. For instance, the rules that translate detected user actions into commands for controlling the medical device 202 may be dependent on the type of alert. If the alert is triggered by a specific condition, the rules may be adjusted to prioritize actions that are commonly associated with responding to that condition. This can ensure that the most relevant actions are recognized and processed first, potentially improving the efficiency of the hands-free control of the medical device 202.

For example, in the case that the hands-free input is a voice input, the hands-free interface 220 may adjust its speech recognition sensitivity based on the identity, role, or proximity of the user 204. For example, if the user 204 is a healthcare provider with a high level of experience, the sensitivity of the speech recognition may be increased to ensure that all relevant commands are detected and processed. Conversely, if the user 204 is a novice or a visitor, the sensitivity of the speech recognition may be decreased to reduce the likelihood of false positives. This user-specific optimization can provide a more personalized and efficient control of the medical device 202, potentially improving the user's experience and the overall effectiveness of the hands-free control system 200.

By way of further example, the hands-free interface may alter an input detection window during which actions of the user are measured/sensed by sensors of the hands-free interface based on characteristics of the alert. That is, an initialization and duration of a detection window may be altered based on information related to the alert. For example, when the hands-free input is a voice input, the hands-free input may adjust a listening window based on the alert characteristics.

In some aspects, the hands-free interface 220 may implement a feedback loop for confirming user commands in specific situations. For instance, if the user 204 issues a command to silence the alert within a certain time window after the alert is activated, the system 200 may provide a feedback prompt asking the user 204 to confirm the command. This can prevent inadvertent silencing of the alert and ensure that the user 204 has sufficient time to assess the situation before silencing the alert. This feedback loop can provide an additional layer of safety and reliability in the hands-free control of the medical device 202, potentially improving the user's experience and the overall effectiveness of the hands-free control system 200.

In some aspects, the hands-free interface 220 may incorporate advanced implementations to enhance the accuracy and reliability of hands-free input detection. For instance, the hands-free interface 220 may implement two action recognition systems with different functionalities and activation methods. The first action recognition system may be a limited one, enabling control of selected alert-related actions such as silencing, delaying, and changing the limits, and being active for a short window. The second action recognition system may provide a larger range of functionalities but may require a trigger word to be active. In some cases, these two recognition systems may be the same system working with different settings during an initial window, and then operating with expanded settings outside the initial window. This dual-system approach can provide a more flexible and adaptive control of the medical device 202, potentially improving the user's experience and the overall effectiveness of the hands-free control system 200.

In some embodiments, the hands-free interface 220 may use multiple sensors from different systems to enhance user location detection and noise removal. For example, the hands-free interface 220 may use microphones from nearby devices, such as other patient monitors, for speech collection. This multi-device sensor data can provide a more comprehensive and accurate detection of user actions, potentially improving the reliability and efficiency of the hands-free control system 200. For instance, if the user 204 is located closer to a nearby patient monitor, the system 200 may prioritize the speech data collected from the microphone of the nearby monitor. This can ensure that the system accurately recognizes the user's commands, even in challenging conditions such as a noisy healthcare environment.

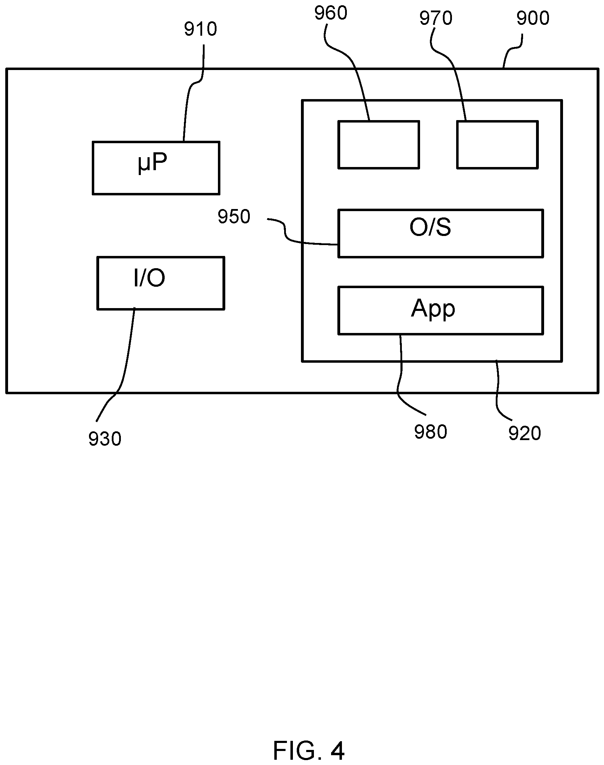

In some cases, the hands-free interface 220 may dynamically select and operate the sensors based on the characteristics of the alert and the detected location of the user 204. For example, if the alert is triggered by a specific condition and the user 204 is located close to the medical device 202, the system 200 may select and operate the sensors that are most suitable for detecting actions commonly associated with responding to that condition in close proximity. This dynamic sensor selection and operation can provide a more adaptive and context-aware control of the medical device 202, potentially improving the user's experience and the overall effectiveness of the hands-free control system 200.FIG. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920 and one or more I/O devices 930 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor-based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs, each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated, the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software stored within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer-related system or method. In the context of this document, a computer readable medium may be an electronic, magnetic, optical, or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s), device(s) and/or system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. In this regard, each block in the flow diagrams or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for detecting a hands-free input from a user for controlling a medical device, the method comprising:
identifying (110) an occurrence of an alert triggered by the medical device; and
detecting (120) the hands-free input from the user, wherein detection of the hands-free input is based in part on one or more characteristics of the alert.

2. The method of claim 1, wherein detecting (120) the hands-free input from the user comprises detecting (124) an action of the user based on the characteristics of the alert.

3. The method of claim 2, wherein detecting (124) an action of the user is initiated responsive to the alert being triggered.

4. The method of claim 3, wherein initiating detection (124) of the action from the user is delayed for a predetermined length of time, and optionally wherein the predetermined length of time is based on at least one of an expected time to review the alert by the user, an expected time for a user to observe the medical device, a length of an output associated with the alert, and/or a user preference.

5. The method of any of claims 2-4, wherein detecting (124) the action of the user is performed during a detection window, wherein the length of the detection window is based on the characteristics of the alert.

6. The method of any of claims 2-5, wherein detecting (124) the action of the user is performed using one or more selected sensors operated according to one or more operation parameters.

7. The method of any of claims 1-6, further comprising detecting (122) the location of the user relative to the medical device, and wherein detection (120) of the hands-free input is further based on the detected location of the user.

8. The method of any of claims 1-7, wherein detecting (120) the hands-free input from the user comprises processing (128), with an action recognition algorithm, a detected action of the user to determine the hands-free input, wherein the processing is based on the characteristics of the alert.

9. The method of claim 8, wherein processing (128) the detected action of the user based on the characteristics of the alert comprises:
modifying (126) the action recognition algorithm based on the characteristics of the alert; and
processing, with the modified action recognition algorithm, the detected action of the user.

10. The method of claim 9, wherein modifying (126) the action recognition algorithm comprises at least one of increasing or decreasing a sensitivity of the action recognition algorithm to user actions, and/or altering a mapping between detected user actions and hands-free inputs by the action recognition algorithm.

11. The method of any of claims 8-10, wherein processing (128) the detected action of the user based on the characteristics of the alert comprises:
filtering the detected action based on the characteristics of the alert; and
processing, with the action recognition algorithm, the filtered detected action of the user.

12. The method of any of claims 8-11, wherein processing (128), with the action recognition algorithm, the detected action of the user to determine the hands-free input is further based on one or more characteristics of the user.

13. The method of any of claims 1-12, wherein the one or more characteristics of the alert include an alert timing, an alert origin, an alert urgency, an alert trigger, an alert location, an alert source, an alert output medium characteristic, an expected user action associated with the alert, and an alert circumstance.

14. A computer program comprising computer program code means adapted, when said computer program is run on a computer, to implement the method of any of claims 1-13.

15. A controller for a medical device (202), the method comprising:
a monitoring system (210) configured to identify an occurrence of an alert triggered by the medical device; and
a hands-free interface (220) configured to detect a hands-free input for controlling the subject-monitor from a user (204), wherein detection of the hands-free input by the hands-free interface is based in part on one or more characteristics of the alert.
